# EUROPEAN PATENT SPECIFICATION

(11) **EP 2 532 315 B1**
(45) Date of publication and mention of the grant of the patent: **05.08.2015**
(21) Application number: 11169450.1
(22) Date of filing: 10.06.2011
(51) Int. Cl.: A61B 17/29

(54) **Medical instrument**
Medizinisches Instrument
Instrument médical

(43) Date of publication of application: 12.12.2012
(73) Proprietor: University of Dundee, Dundee DD1 4HN (GB)
(72) Inventor: Coleman, Stuart, Dundee DD2 1FD (GB); Frank, Timothy Graham, Wormit Newport-On-Tay Fife DD68NG (GB)
(74) Representative: Witte, Weller & Partner Patentanwälte mbB

(56) References cited:
- EP-A1- 1 872 729
- DE-U1- 9 307 793
- US-A- 5 607 449
- US-A- 5 643 294

## Description

The invention relates to a medical instrument, comprising an elongated shaft having a distal end and a proximal end, a working part arranged at the distal end of the shaft, a handle arranged at the proximal end of the shaft, and an elongated element extending through the shaft, a distal end of the elongated element being rotationally fixedly connected to the working part and a proximal end of the elongated element being connected to the handle, the shaft being rotatable relative to the handle about a longitudinal axis of the shaft, the working part being rotatable relative to the handle about a longitudinal axis of the working part independently of the shaft, and an actuating element arranged on the handle for rotating the shaft.

A medical instrument of the kind mentioned before is known from EP 1 872 729 B1.

A medical instrument according to the present invention can be designed as an instrument for grasping tissue, as an instrument for cutting tissue, as an instrument for use in electrosurgery (coagulation and cutting with high frequency currency), as a needle holder, a dissector, for example.

A medical instrument according to the invention is particularly designed for use in endoscopic surgery.

The present invention is particularly useful, if the shaft of the instrument has at least one bent portion between the distal end and the proximal end, wherein, however, the present invention is not limited thereto. In case that the shaft has bent portion, the bent portion can have at least one fixed curve or an articulating joint. The invention can be applied in either case.

When using a medical instrument having a shaft with at least one bent portion, it may be desirable for the working part of the instrument to be oriented at different angles to the shaft and/or the handle of the instrument. Allowing the working part to rotate independently of the shaft enables additional movements to be carried out by the instrument, i.e. the orientation of the curved shaft may be altered without changing the working part angle and vice versa.

The shaft and the working part of the medical instrument known from EP 1 872 729 B1 are rotatable relative to the handle independently of one another. In order to control the rotation of the shaft, the known medical instrument has a first actuating element, and for controlling the rotation of the working part, the known medical instrument has a second actuating element. Both actuating elements are arranged on the handle. Such an arrangement of two actuating elements may not be ideal as only one actuating element can be placed in the optimal ergonomic position for one hand operation. Furthermore, if two actuating elements are used and it is desired to rotate both the working part and the shaft simultaneously, then it may be difficult for the user to actuate both actuating elements together.

DE 196 47 761 C1 discloses a medical instrument which has a first actuating element which is axially displaceable and rotatable about the longitudinal axis of the shaft in order to control the curvature of a bent distal portion of the shaft. The axial movement of the actuating element serves for roughly adjusting the curvature, and the rotation of the actuating element serves as a fine adjustment of the curvature. A second actuating element is arranged adjacent to the first actuating element for rotating the working part at the distal end of the shaft. Again, this known medical instrument has at least two actuating elements in order to provide different orientations of the working part at the distal end.

It is, therefore, an object of the present invention to improve a medical instrument of the type mentioned at the outset such that the rotational movements of the working part and the shaft can be actuated by the user with improved ergonomy.

This object is achieved with respect to the medical instrument of the type mentioned at the outset in that the actuating element is switchable between at least two different axial positions, in a first position of which the actuating element is rotationally fixedly coupled exclusively with the shaft for rotating the shaft only, in a second position of which the actuating element is rotationally fixedly coupled exclusively with the elongated element for rotating the working part only.

The medical instrument according to the invention, thus, comprises an actuating element which can control both, the rotational movement of the shaft and the rotational movement of the working part. This is achieved by virtue of the fact that the actuating element can be switched between at least two axial positions, in order to, on the one hand, rotationally fixedly couple the actuating element with the shaft, and, on the other hand, to rotationally fixedly couple the actuating element with the elongated element and, thus, with the working part. In other words, the medical instrument according to the invention has a coupling between the actuating element and the shaft and the elongated element, which can be selectively switched by axially moving the actuating element. Since only one actuating element is necessary in order to control the rotational movement of the shaft and the rotational movement of the working part, the ergonomy of the medical instrument is improved, while the high number of degrees of freedom of movement of the working part is maintained.

The actuating element of the medical instrument according to the invention is preferably a control wheel arranged on the handle which is rotatable about the longitudinal axis of the shaft in order to control the rotation of the working part and the rotation of the shaft. It is, however, conceivable to design the actuating element as a lever rotatable about the longitudinal axis of the shaft, for example.

In a preferred refinement, the actuating element is switchable into a third axial position, in which the actuating element is rotationally fixedly coupled simultaneously with the shaft and the elongated element for rotating both the shaft and the working part simultaneously.

In this refinement, the actuating element cannot only control the rotational movement of the shaft and of the working part independently of one another, but the actuating element can be switched in another axial position for controlling the simultaneous rotational movement of the shaft and of the working part. If two actuating elements are used, as it is the case with the known medical instrument, then it may be difficult for the user to actuate both actuating elements synchronously. In contrast, the present refinement enables a synchronous rotation of the shaft and of the working part in an ergonomical way.

In further preferred refinements, the actuating element is rotationally fixedly coupled with the shaft and/or with the elongated element by positive locking engagement, and/or by frictional engagement.

A coupling between the actuating element and the shaft and/or the elongated element which is based on positive locking engagement has the advantage that the coupling is locked securely so that it is ensured that an actuation of the actuating element is transferred into a rotational movement of the shaft and/or the elongated element in any case. Thus, the coupling is fail-safe.

A coupling between the actuating element and the shaft and/or the elongated element based on frictional engagement has the advantage that structurally simple elements can be used as the coupling and, using frictional elements to establish the coupling between the actuating element and the shaft and/or the elongated element can further provide a self-inhibiting effect in order to lock that component (shaft or elongated element) against rotation when this component is not actually controlled by the actuating element.

In a preferred refinement which uses a coupling between the actuating element and the shaft and/or the elongated element mainly based on positive locking engagement, the actuating element is rotationally fixedly coupleable with the shaft and/or the elongated element via sets of splines extending in longitudinal direction, a first set of splines being rotationally fixed with respect to the shaft, a second set of splines being rotationally fixed with respect to the elongated element, at least one further spline being rotationally fixed with respect to the actuating element.

Such splines can be designed as ribs or grooves on bushings, a first bushing being rotationally fixed with respect to the shaft and a second bushing being rotationally fixed with respect to the elongated element, or on integral parts of the shaft or the elongated element, while the at least one further spline which is rotationally fixed with respect to the actuating element can be a single rib or groove cooperating with the first and second sets of splines in meshing fashion. It is, however, preferred if a plurality of further splines is rotationally fixed with respect to the actuating element, for example in case that the actuating element is configured as a control wheel, on the inner surface of the control wheel.

According to a further preferred refinement, coupling between the actuating element and the shaft and/or the elongated element is accomplished by axially displacing the at least one further spline so that the at least one further spline meshes with the first set of splines and/or with the second set of splines.

In connection with one of the above-mentioned refinements, according to which the actuating element has a third position for simultaneously controlling the rotation of the shaft and the elongated element, the at least one further spline has a sufficient length in longitudinal direction so that it can simultaneously mesh with the first set of splines and the second set of splines.

In a further preferred refinement, the splines of the first set of splines and the splines of the second set of splines are circumferentially distributed around the longitudinal axis.

This measure has the advantage that coupling the actuating element with the shaft and/or the elongated element is possible in many rotational orientations of the shaft and/or the elongated element. The more splines are circumferentially distributed around the longitudinal axis, the less important is the relative rotational orientation of the actuating element with respect to the first set of splines and/or the second set of splines, in order to be able to engage.

In a further preferred refinement, the splines of the first set of splines, the splines of the second set of splines and/or the at least one further spline have tapered ends.

This measure has the advantage that it is ensured that the at least one further spline can engage the splines of the first set of splines and/or the splines of the second set of splines from any angular orientation about the longitudinal axis.

In a further preferred refinement, the first set of splines is arranged adjacent to the second set of splines with no or at least only low frictional contact to one another.

This measure advantageously prevents that one component (shaft or elongated element) which is not actually controlled by the actuating element from being entrained to rotation, when the other component is rotated. For example, if the actuating element actually controls the elongated element, the afore-mentioned measure avoids that the shaft is rotated. An additional or alternative measure to prevent such an undesired entrainment may be to provide the first set of splines and/or the second set of splines with reasonably tight fit inside the handle or via a part which is sprung against the handle in order to produce friction.

In a further preferred refinement of the coupling between the actuating element and the shaft and/or the elongated element which is based on frictional engagement and/or positive locking engagement provides that the actuating element is rotationally fixedly coupleable with the shaft and/or the elongated element via radially elastically deformable elements, a first elastically deformable element being rotationally fixed with respect to the shaft and a second elastically deformable element being rotationally fixed with respect to the elongated element.

The use of elastically deformable elements as the coupling between the actuating element and the shaft and/or the elongated element has the advantage that such elastically deformable elements cannot only provide a rotationally fixed coupling, but also a locking of that component (shaft or elongated element) that is not actually controlled by the actuating element.

In a preferred refinement of the afore-mentioned refinement, the first elastically deformable element and the second elastically deformable element each have at least one leaf spring, respectively, extending in longitudinal direction, a respective first end of each leaf spring being fixed with respect to the shaft and the elongated element, respectively, and a respective second end of each leaf spring being free and biased in radial direction away from the longitudinal axis, wherein when slid over the respective second end of the respective leaf spring, the actuating element urges the second end in direction towards the longitudinal axis, the respective second end thereby being in rotationally fixed engagement with the actuating element.

In this refinement, the coupling between the actuating element and the shaft and/or the elongated element thus uses leaf springs which are structurally simple parts which are easily to be manufactured. Further, elastically deformable elements using leaf springs provide a coupling between the actuating element and the shaft and/or the elongated element which can solely be based on frictional engagement. A coupling based on frictional engagement has the advantage that the actuating element can engage the elastically deformable elements from any angular position about the longitudinal axis, i.e. there are no distinct angular positions about the longitudinal axis at which engagement is possible, but there is a continuum of all angular positions at which the actuating element can engage the leaf springs.

While one leaf spring for the first and second elastically formable element is basically sufficient, it is, however, preferable if the first elastically deformable element and the second elastically deformable element each have a plurality of leaf springs circumferentially distributed about the longitudinal axis. In particular, if the second ends of the leaf springs only frictionally engage the actuating element, this measure has the advantage that sufficient friction is produced in order to ensure a rotational locking between the actuating element and the shaft and/or the elongated element.

In a further preferred refinement, the at least one leaf spring of the first elastically deformable element and the at least one leaf spring of the second elastically deformable element are oppositely inclined with respect to the longitudinal axis, wherein the second ends of the leaf springs are facing away from one another.

This measure is particularly useful if the actuating element has a third position in which the actuating element is rotationally fixed with the shaft as well as rotationally fixed with the elongated element. For, in this case the third axial position of the actuating element is maintained because the actuating element is balanced between the oppositely inclined leaf springs which are both forcing the actuating element in opposite axial directions. It is, thereby, not necessary to provide a latching mechanism, for example, in order to ensure that the actuating element rests in the third position.

Further, the first position and the second position of the actuating element can be secured according to a preferred refinement which provides that the actuating element has at least one radially inwardly directed protrusion behind which the respective second end of the respective leaf spring comes to lie when in engagement with the actuating element.

In addition to the advantage that the first and/or second position of the actuating element is secured by this measure, a further advantage of this measure is that the user can tactilely recognize that the first or the second position of the actuating element is secured when the respective second end of the respective leaf spring springs behind the radially inwardly directed protrusion.

The at least one protrusion can extend over the full circumference about the longitudinal axis, but can also extend over a part of the circumference only. Preferably, there are two protrusions, one for the first position and one for the second position of the actuating element.

In a further preferred refinement, the at least one leaf spring of the first elastically deformable element is in rotationally fixed engagement with the handle, when the actuating element is in the second position, and the at least one leaf spring of the second elastically deformable element is in rotationally fixed engagement with the handle, when the actuating element is in the first position.

This measure has the advantage that the first elastically deformable element and the second elastically deformable element do not only have the function to rotationally fixedly couple the actuating element with the shaft and/or the elongated element, but also to rotationally lock the shaft and/or the elongated element (and thus the working part) to the handle if not actually rotationally fixedly coupled with the actuating element.

The working part of the medical instrument according to the invention can have at least one moveable working element, for example the working part can have two jaw parts, one of which or both is or are moveable, in order to provide a cutting or grasping action, for example. In this case, it is provided in a further refinement that the elongated element is axially moveable with respect to the second set of splines or that the elongated element is axially moveable with respect to the second elastically deformable element.

Thus, it is to be acknowledged that the present invention cannot only be used for instruments having working parts which do not have moveable working elements, but also for medical instruments which have working parts with moveable working elements.

Further, as already mentioned above, the present invention is particularly useful if the shaft of the medical instrument has at least one bent portion, which can have at least one fixed curve or at least one articulating joint.

Further features and advantages will become apparent from the following description and the drawings.

It is to be understood that the afore-mentioned features and those features to be described below are not only applicable in the given combination, but also in other combinations or in isolation without departing from the scope of the present invention.

Exemplary embodiments are shown in the drawings and will be described hereinafter with respect thereto. In the drawings:
Figure 1 shows a medical instrument in a side view;
Figure 2 shows the instrument of figure 1 in a side view and in enlarged scale, wherein a shaft of the instrument is shown partially;
Figure 3 shows a handle portion of the instrument of figures 1 and 2 in the region of an actuating element in a longitudinal sectional view and in further enlarged scale;
Figure 4 shows the handle portion in figure 3, additionally in a cross-sectional view taken along a line IV-IV in figure 3 and further in a perspective view;
Figure 5 shows the handle portion in figure 3, wherein the actuating element is shown in an operating state different from the operating state of the actuating element in figure 3;
Figure 6 shows the handle portion in figure 3, wherein the actuating element is shown in an operating state different from the operating states in figures 3 and 5;
Figure 7 shows another embodiment of the handle portion of the instrument in figures 1 and 2 in a longitudinal sectional view;
Figure 8 shows the handle portion in figure 7, wherein an actuating element of the handle portion is in a different operating state than in figure 7; and
Figure 9 shows a detail of the handle portion in figures 7 and 8.

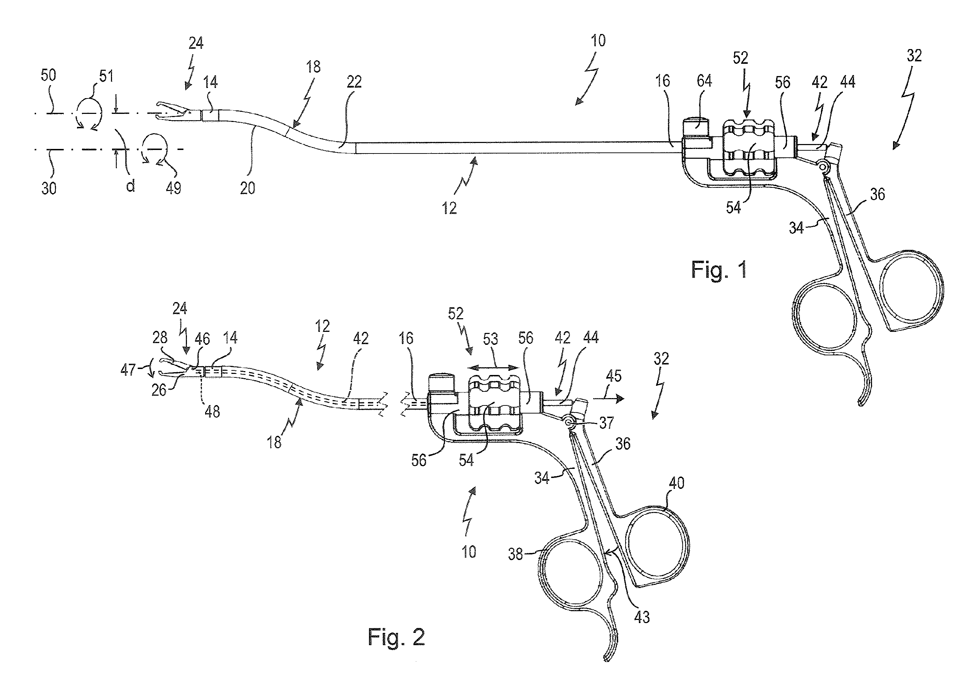

Figs. 1 and 2 show a medical instrument labelled with general reference numeral 10. The medical instrument 10 is configured as a forceps, in particular for use in endoscopic surgery.

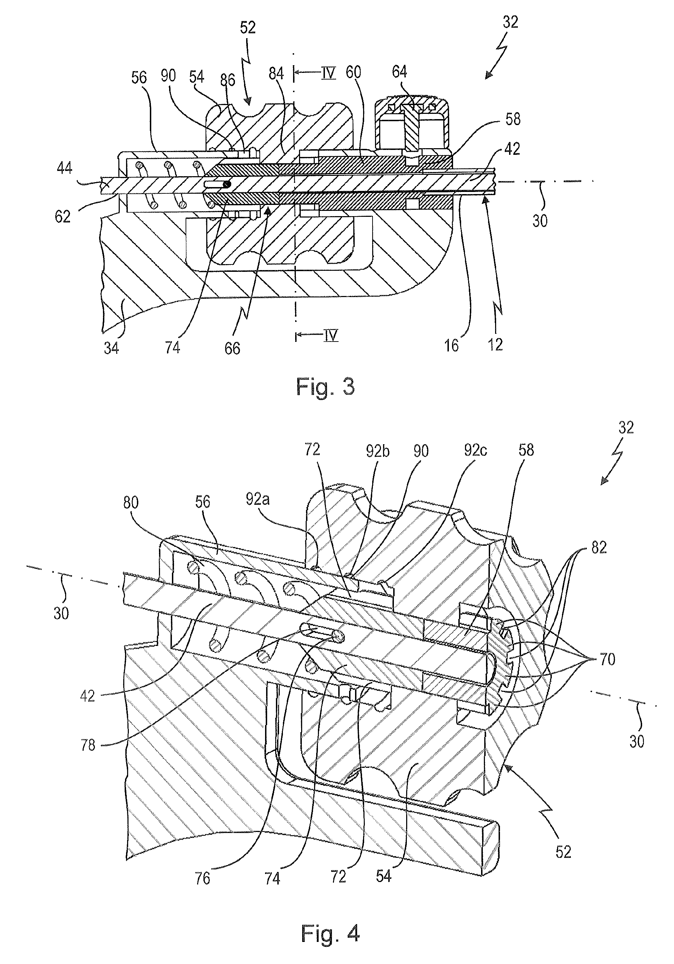

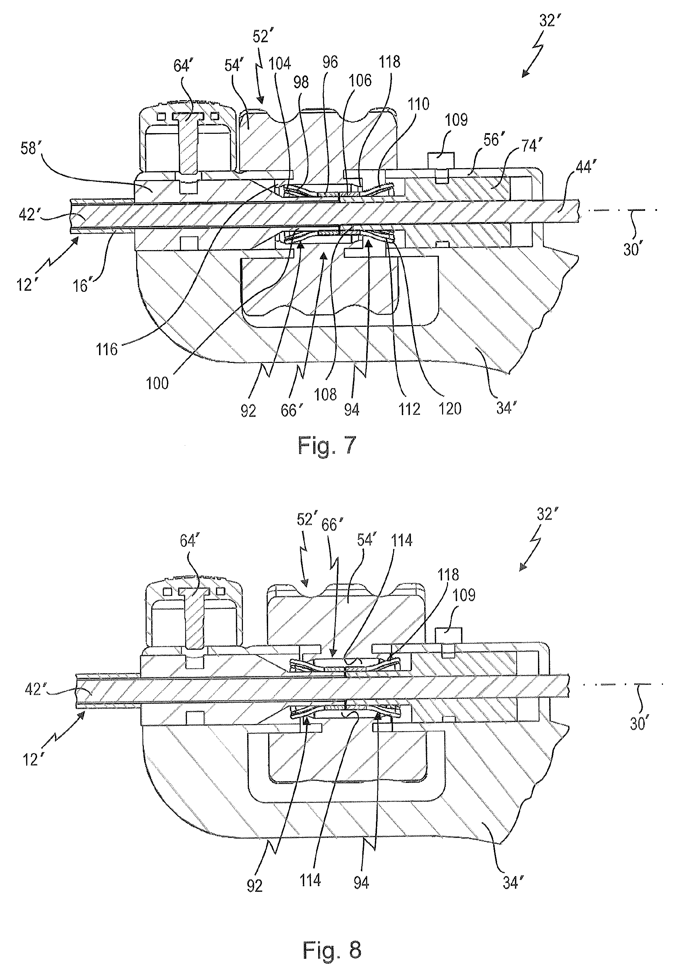

Further details of the instrument 10, according to a first embodiment, are shown in figs. 3 through 6, and, according to a second embodiment, in figs. 7 through 9.

With reference to figs. 1 and 2, the instrument 10 comprises an elongated shaft 12 which has a distal end and a proximal end 16.

The shaft 12 has at least one bent portion 18 between the distal end 14 and the proximal end 16. In the present embodiment, the bent portion 18 is a so-called bayonet bent portion having two curvatures 20 and 22 having their center of curvature on opposite sides of the shaft 12. Thus, the bent portion 18 resembles an S-shape so that the distal end 14 and the proximal end 16 are substantially parallel with one another.

It is to be understood that the bent portion 18 could also have only one curvature, for example the curvature 22 so that in this case the distal end 14 and the proximal end 16 form an angle with one another.

The bent portion 18 may be permanent if the shaft 12 is rigid, wherein the bent portion 18 has been shaped when manufacturing the shaft 12. It is, however, also possible that the shaft 12 is semi-rigid or semi-flexible so that the bent portion 18 can be shaped by the user of the instrument 10 by bending the shaft 12 into a desired shape.

Furthermore, the term "bent portion" according to the invention can also encompass a configuration of the shaft 12 where two portions of the shaft are articulated with one another via a joint. It is to be understood that the shaft 12 may have more than one bent portion.

A working part 24 is arranged at the distal end 14 of the shaft 12. The working part 24 has an immovable working element 26 and a movable working element 28. The working elements 26 and 28 are configured as jaw parts for grasping, for example, tissue. In the embodiment shown, the medical instrument 10, thus, is a grasping instrument.

Due to the bent portion 18, the working part 24 is laterally displaced with respect to a longitudinal axis 30 of the shaft 12 (the longitudinal axis 30 coincides with the longitudinal axis of the proximal end 16) by a distance d (see fig. 1).

A handle 32 is arranged at the proximal end 16 of the shaft 12. The handle 32 has an immovable grip part 34 and a movable grip part 36. The immovable grip part 34 has a finger ring 38, and the movable grip part 36 has a finger ring 40. The grip parts 34 and 36 are configured in the fashion of scissors grips.

The movable grip part 36 is operatively connected with the movable working element 28 via an elongated element 42, wherein the elongated element 42 extends through the shaft 12 (as indicated by broken lines in fig. 2) so that only a proximal end 44 of the elongated element 42 can be seen in figs. 1 and 2.

The elongated element 42 is configured as a force transmission element in the embodiment shown and is axially movable with respect to the shaft 12 in order to transfer a movement of the movable grip part 36 which is pivotable about a hinge 37, into a movement of the movable working element 28 for closing and opening the working part 24. A clockwise pivoting (see arrow 43 in fig. 2) of the movable grip part 36 about a pivoting axis of the hinge 37 which is perpendicular to the plane of drawing in fig. 2 retracts the elongated element 42 according to an arrow 45 in fig. 2, which retraction of the elongated element 42 causes the movable working element 28 to pivot about a pivoting axis of a hinge 46 in counter-clockwise direction according to an arrow 47 in fig. 2 towards the immovable working element 26. The movable working element 28 is pivoted away from the immovable working element 26 by pivoting the movable grip part 36 in counter-clockwise direction about the pivoting axis of the hinge 37, accordingly.

A distal end 48 of the elongated element 42 is rotationally fixedly connected to the working part 24, i.e. more exactly to the movable working element 28 of the working part 24.

The shaft 12 is rotatable about the longitudinal axis 30 relative to the handle 32 in clockwise and counter-clockwise direction according to an arrow 49.

The working part 24 is also rotatable about a longitudinal axis 50 (see fig. 1) of the working part 24 in clockwise and counter-clockwise direction according to an arrow 51. Since the working part 24 is rotationally fixedly connected to the elongated element 42, the working part 24 rotates together with the elongated element 42 relative to the handle 32.

In particular, the working part 24 and the shaft 12 are rotatable relative to the handle independently of one another, i.e. the working part 24 can be rotated about the longitudinal axis 50 while the shaft 12 does not rotate about the longitudinal axis 30 and vice versa. Further, the working part 24 and the shaft 12 can also be rotated simultaneously, i.e. while the shaft 12 is rotated about the longitudinal axis 30, the working part 24 is simultaneously rotated about the longitudinal axis 50. It is to be understood that the longitudinal axis 50 revolves about the longitudinal axis 30 when the shaft 12 is rotated.

In order to control the rotation of the working part 24 relative to the handle 32 while the shaft 12 does not rotate, or to control the rotation of the shaft 12 while the working part 24 does not rotate, or for simultaneously rotating the working part 24 and the shaft 12, there is only one actuating element 52 arranged in a position on the handle 32, which is ergonomical for actuation by the index finger of the same hand holding the handle 32.

The actuating element 52 is configured as a control wheel 54 which is rotatable about the longitudinal axis 30 of the shaft 12. Further, in order to enable rotation of the shaft 12 only, of the working part 24 only, and of the shaft 12 and the working part 24 simultaneously, the actuating element 52 is displaceable in axial direction, i.e. in direction of the longitudinal axis 30 according to an arrow 53. Figs. 1 and 2 show the actuating element 52 in its most proximal position, from which the actuating element 52 can be axially displaced in distal direction.

By axially displacing the actuating element 52, the actuating element 52 can be switched between three axial positions, wherein in a first position the actuating element 52 is rotationally fixedly coupled exclusively with the shaft 12 for rotating the shaft 12 only, in a second position the actuating element 52 is rotationally fixedly coupled exclusively with the elongated element 42 for rotating the working part 24 only, and in a third position the actuating element 52 is rotationally fixedly coupled simultaneously with the shaft 12 and the elongated element 42 for rotating both the shaft 12 and the working part 24 simultaneously. The afore-mentioned selective coupling of the actuating element 52 with the shaft 12 only, the elongated element 42 only and both the shaft 12 and the working element 42 simultaneously will be described hereinafter.

Figs. 3 through 6 show a portion of the medical instrument 10 in the region of the handle 32. The handle has a handle housing 56 having a tubular shape. The actuating element 52 in form of the control wheel 54 is placed around the handle housing 56. The shaft 12, which is shown in fig. 3 in the region of its proximal end 16 has a proximal extension 58 which is arranged in the handle housing 56 with reasonably tight fit to an inner surface 60 of the handle housing 56.

The elongated element 42 extends through the proximal extension 58 of the shaft 12 and through the handle housing 56, wherein the proximal end 44 of the elongated element 42 extends through an opening 62 at the proximal end of the handle housing 56.

A fixing screw 64 secures the proximal extension 58 of the shaft 12 to the handle 32, wherein the proximal extension 58 and, thus, the shaft 12 are only axially secured by the fixing screw 64, but not rotationally.

The actuating element 52 is selectively coupleable with the elongated element 42 and/or the shaft 12 via a coupling 66 which is based on positive locking engagement of the actuating element 52 with the shaft 12 and/or the elongated element 42.

The coupling 66 comprises a first set of splines 70 (see fig. 4) which are configured in form of ribs which are circumferentially distributed around the proximal end of the proximal extension 58 of the shaft 12. The first set of splines 70 is rotationally fixed with respect to the shaft 12, which is, in the present embodiment, accomplished by the fact that the first set of splines 70 is integrally formed on the proximal extension 58, which in turn is integrally formed with the shaft 12. The splines 70 extend in direction of the longitudinal axis 30. The splines 70 are distributed over the full circumference of the proximal extension 58 of the shaft 12.

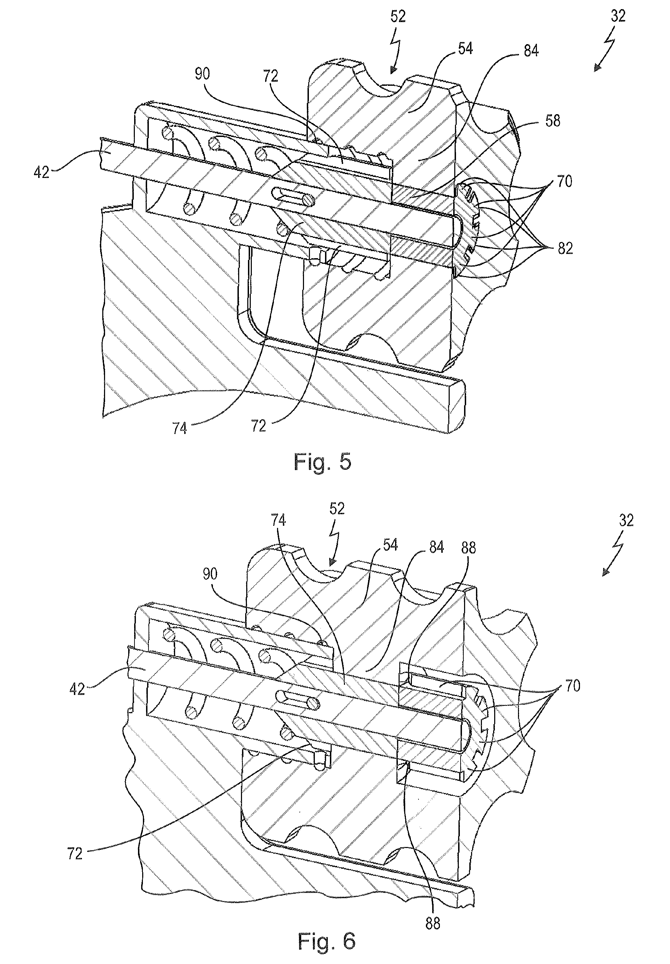

A second set of splines 72 (see figs. 4 and 5) is rotationally fixed with respect to the elongated element 42. The second set of splines 72 which also extend in direction of the longitudinal axis 30 is arranged on a connector 74 which is configured as a bushing through which the elongated element 42 extends. The connector 74 and, thus, the second set of splines 72 is rotationally fixed with respect to the elongated element 42 by a pin 76 arranged in an oblong hole 78 formed in the elongated element 42. Thus, the elongated element 42 is axially movable with respect to the connector 74. The connector 74 is axially fixed to the handle 32 by a spring 80 which pushes the connector 74 in distal direction, but rotatable relative to the handle 32.

The splines 72 are circumferentially distributed about the outer surface of the connector 74 and are configured as ribs of the same shape as the splines 70.

A third set of splines 82 (see fig. 5) is rotationally fixed to the actuating element 52, i.e. to the control wheel 54. The splines 82 which are formed as grooves extend in direction of the longitudinal axis 30. The splines 82 are complementary to the splines 70 and 72 as can be seen in fig. 5.

The splines 82 are integrally formed on a flange 84 which is integrally formed with the control wheel 54 and protrudes radially inwardly through a circumferential opening in the handle housing 56.

The third set of splines 82 is configured to mesh with the first set of splines 70 and/or the second set of splines 72, wherein the meshing engagement is determined by the axial position of the actuating element 52 in direction of the longitudinal axis 30.

Fig. 3 and 4 show an axial position of the actuating element 52 in which the third set of splines 82 meshes with the first set of splines 70 as well as with the second set of splines 72. In this axial position, the actuating element 52 is rotationally fixedly coupled with the shaft 12 as well as with the elongated element 42. Actuating the actuating element 52 by rotating same about the longitudinal axis 30 thus causes the shaft 12 and the elongated element 42 and, thus, the working part 24 to rotate simultaneously, wherein the shaft 12 is rotated about the longitudinal axis 30 and the working part 24 is rotated about the longitudinal axis 50 (see fig. 1).

By axially displacing the actuating element 52 in proximal direction as shown in fig. 6, the third set of splines 82 only meshes with the second set of splines 72 so that the actuating element 52 is rotationally fixedly coupled with the elongated element 42 only. In this position of the actuating element 52, rotating the control wheel 54 about the longitudinal axis 30 causes the elongated element 42 and, thus, the working part 24 to rotate about the longitudinal axis 50 while the shaft 12 does not rotate about the longitudinal axis 30.

Vice versa, if the actuating element 52 is displaced in the position shown in fig. 5 in distal direction, the third set of splines 82 only meshes with the first set of splines 70. In this case, rotating the control wheel 44 about the longitudinal axis 30 only causes the shaft 12 to rotate about the longitudinal axis 30, while the elongated element 42 and, thus, the working part 24 does not rotate about the longitudinal axis 50.

While the third set of splines comprises a plurality of splines 82, it is also conceivable that the actuating element 52 only has one of the splines 82 which is sufficient in order to rotationally fixedly couple the actuating element 52 with the shaft 12 and/or the elongated element 42.

As can be seen in fig. 6, the splines 70 have tapered ends 88. The splines 72 and 82 have tapered ends, accordingly. The ends 88 taper in circumferential direction about the longitudinal axis 30. It is to be understood that the splines 70, 72 and 82 are tapered at least at their ends facing one another, respectively. The tapered ends 88 ensure that the splines 82 can engage the splines 70 and/or 72 from any angular orientation with respect to the longitudinal axis 30.

Further, as shown in figs. 3 through 6, the first set of splines 70 is arranged adjacent to the second set of splines 72, wherein it is preferred if the first set of splines 70 and the second set of splines 72 are arranged with no or at least only low frictional contact to one another in order to ensure that that set of splines 70 or 72 which not actually meshes with the third set of splines 82 is not entrained by a rotation of the control wheel 54.

Further, the connector 74 and the proximal extension 58 can be provided with a reasonably tight fit inside the handle housing 56 or via a part which is sprung against the handle housing 56 in order to produce sufficient friction.

The three axial positions of the actuating element 52 explained above can be defined by a latching mechanism, which, for example, can comprise a ball spring 90 (see fig. 3), wherein the ball of the ball spring 90 is arranged on the outer surface of the handle housing 56, and which cooperates with three grooves 92a, 92b, 92c formed in an inner surface of the actuating element 52.

Figs. 7 and 8 show another embodiment of a coupling for selectively rotationally fixedly coupling the actuating element with the shaft and/or the elongated element. Those components shown in figs. 7 and 8 which are identical to, similar to or comparable with corresponding parts in figs. 3 through 6 are labelled with the same reference numerals as in figs. 3 through 6 supplemented by a '.

In the following, the differences between the embodiment shown in figs. 7 and 8 with respect to the embodiment shown in figs. 3 through 6 will be described.

The actuating element 52' is rotationally fixedly coupleable with the shaft 12' and/or the elongated element 42' by a coupling 66' which is based on frictional engagement.

The coupling 66' comprises a first radially elastically deformable element 92 and a second radially elastically deformable element 94.

The first deformable element 92 is rotationally fixed with respect to the shaft 12' and the second deformable element 94 is rotationally fixed with respect to the elongated element 42'.

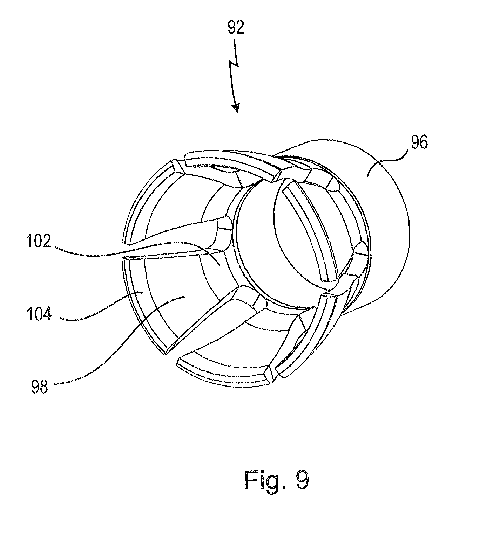

The first deformable element 92 is shown in isolation and in a perspective view in fig. 9. The same description applies to the second deformable element 94.

The first deformable element 92 has a sleeve portion 96 and a plurality of leaf springs 98. In the present embodiment, the deformable element 92 has six leaf springs 98.

With reference to fig. 7, the sleeve portion 96 is fixed to a further extension 100 of the proximal extension 58' of the shaft 12' so that the deformable element 92 is rotationally fixed with respect to the shaft 12'. The leaf springs 98 extend in direction of the longitudinal axis 30' and are inclined with respect thereto.

A first end 102 of each leaf spring 98 which departs from the sleeve portion 96 is fixed with respect to the shaft 12', and a second end 104 of each leaf spring 98 is free and biased in radial direction away from the longitudinal axis 30'.

The second deformable element 94 which is configured identical to the first deformable element 92 has its sleeve portion 106 fixed to an extension 108 of the connector 74' to which the elongated element 42' is connected so as to be axial displaceable with respect to the connector 74' but rotationally locked.

As can be seen in figs. 7 and 8, the leaf springs 110 of the second deformable element 94 and the leaf springs 98 of the first deformable element 92 are oppositely inclined with respect to the longitudinal axis 30', wherein the second ends 104 of the leaf springs 98 and the second ends 112 of the second deformable element 94 face away from one another.

The connector 74' is axially fixed, but not rotationally fixed with respect to the handle housing 56' by a fixing screw 109.

The actuating element 52' has a radially inner surface 114 which is spaced apart from the longitudinal axis 30' in a shorter distance than the distance of the second ends 104 and 112 from the longitudinal axis 30', when the latter are not depressed. The axial length in direction of the longitudinal axis 30' of the inner surface 114 is shorter than the distance between the second ends 104 and the second ends 112 of the deformable elements 92 and 94.

The actuating element 52' has a radially inwardly directed protrusion 116 at one axial end of the surface 114, and another radially inwardly directed protrusion 118 at the other axial end of the surface 114. The protrusions 116 and 118 extend circumferentially about the longitudinal axis 30'.

Fig. 7 shows the actuating element 52' in an axial position in direction of the longitudinal axis 30' in which the actuating element 52' is slid over the second ends 104 of the leaf springs 98 of the first deformable element 92, whereby the actuating element 52', i.e. the inner surface 114 urges the second ends 104 in direction towards the longitudinal axis 30', whereby the second ends 104 are in rotationally fixed engagement with the actuating element 52'. This engagement can be purely frictional, but can also be a positive locking engagement, wherein in the latter case suited features are provided on the inner surface 114 and/or on the second ends 104 of the leaf springs 98 in order to provide a positive locking between these elements.

In the position of the actuating element 52' shown in fig. 7, the second ends 112 of the second deformable element 94 are not in engagement with the actuating element 52', but rest against the handle housing 56' as shown with reference numeral 120 in fig. 7, whereby the elongated element 42' is rotationally fixed with respect to the handle 32'. Rotating the control wheel 54' in the position of the actuating element 52' in fig. 7 causes the shaft 12' to rotate about the longitudinal axis 30', while the elongated element 42' does not rotate, and, thus, the working part 24 does not rotate about the longitudinal axis 50.

Further, as can be seen in fig. 7, the second ends 104 of the leaf springs 98 rest behind the protrusion 116 of the surface 114, whereby the axial position of the actuating element 52' is secured. This ensures that the actuating element 52' will stay in its axial position until it is deliberately moved.

By axially displacing the actuating element 52' from the position shown in fig. 7, the actuating element 52' reaches the position shown in fig. 8. In this position, the leaf springs 98 of the first deformable element 92 and the leaf springs 110 of the second deformable element 94 are both in engagement with the actuating element 52', wherein this engagement is established between the protrusions 116 and 118 and the leaf springs 98 and 110. In this position, the actuating element 52' is rotationally fixedly coupled with both deformable elements 92 and 94, so that a rotation of the control wheel 94' about the longitudinal axis 30' will cause the shaft 12' and the elongated element 42' and thus the working part 24 to rotate simultaneously.

In the position of the actuating element 52' shown in fig. 8, the actuating element 52' is axially secured, because the actuating element 52' is balanced between the leaf springs 98 and the leaf springs 110 which are both forcing the actuating element 52' axially in opposite directions.

From the position of the actuating element 52' shown in fig. 8 the actuating element 52' can be further axially displaced in proximal direction (right hand side direction in fig. 8), thereby further urging the leaf springs 110 in radial direction towards the longitudinal axis 30' until the protrusion 118 springs over the second ends 112 of the leaf springs 110, whereby the second deformable element 94 is rotationally fixedly coupled with the actuating element 52', while the leaf springs 98 are clear of the actuating element 52' and rest against the handle housing 56'. In this position, a rotation of the control wheel 54' causes the elongated element 42' and thus the working part 24 to rotate only, while the shaft 12' is rotationally fixed with respect to the handle 32'.

The elongated element 42' is axially movable with respect to the connector 74' in order to be able to serve as a force transmission element for moving axially the movable working element 28 according to fig. 1.

In the present embodiment, the deformable elements 92 and 94 serve three purposes, namely to lock the shaft 12' or the elongated element 42' when the shaft 12' or the elongated element 42' is not being controlled by the actuating element 52', to provide a rotationally fixed coupling between the actuating element 52' and the shaft 12' and/or the elongated element 42', and to provide three distinct axial positions into which the actuating element 52' may be moved and in which it is secured in each case. The actuating element 52' may only rest in one of these three positions, and a moderate force will be required to switch the actuating element 52' between these positions.

## Claims

1. A medical instrument, comprising an elongated shaft (12; 12') having a distal end (14) and a proximal end (16; 16'), a working part (24) arranged at the distal end (14) of the shaft (12; 12'), a handle (32; 32') arranged at the proximal end (16; 16') of the shaft (12; 12'), and an elongated element (42; 42') extending through the shaft (12; 12'), a distal end (48) of the elongated element (42; 42') being rotationally fixedly connected to the working part (24) and a proximal end (44; 44') of the elongated element (42; 42') being connected to the handle (32; 32'), the shaft (12; 12') being rotatable about a longitudinal axis (30; 30') of the shaft (12; 12'), the working part (24) being rotatable relative to the handle (32; 32') about a longitudinal axis (50) of the working part (24) independently of the shaft (12; 12'), and an actuating element (52; 52') arranged on the handle (32; 32') for rotating the shaft (12; 12'), **characterized in that** the actuating element (52; 52') is switchable between at least two different axial positions, in a first position of which the actuating element (52; 52') is rotationally fixedly coupled exclusively with the shaft (12; 12') for rotating the shaft (12; 12') only, and in a second position of which the actuating element (52; 52') is rotationally fixedly coupled exclusively with the elongated element (42; 42') for rotating the working part (24) only.

2. The medical instrument of claim 1, **characterized in that** the actuating element (52; 52') is switchable into a third axial position, in which the actuating element (52; 52') is rotationally fixedly coupled simultaneously with the shaft (12; 12') and the elongated element (42; 42') for rotating both the shaft (12; 12') and the working part (24) simultaneously.

3. The instrument of claim 1 or 2, **characterized in that** the actuating element (52; 52') is rotationally fixedly coupleable with the shaft (12; 12') and/or with the elongated element (42; 42') by positive locking engagement.

4. The instrument of any one of claims 1 through 3, **characterized in that** the actuating element (52') is rotationally fixedly coupled with the shaft (12') and/or with the elongated element (42') by frictional engagement.

5. The instrument of any one of claims 1 through 4, **characterized in that** the actuating element (52) is rotationally fixedly coupleable with the shaft (12) and/or the elongated element (42) via sets of splines (70, 72, 82) extending in longitudinal direction, a first set of splines (70) being rotationally fixed with respect to the shaft (12), a second set of splines (72) being rotationally fixed with respect to the elongated element (42), at least one further spline (82) being rotationally fixed with respect to the actuating element (52).

6. The instrument of claim 5, **characterized in that** the actuating element (52) is coupleable with the shaft (12) and/or the elongated element (42) by axially displacing the at least one further spline (82) so that the at least one further spline (82) meshes with the first set of splines (70) and/or with the second set of splines (72).

7. The instrument of claim 5 or 6, **characterized in that** the splines (70) of the first set of splines (70) and the splines (72) of the second set of splines (72) are circumferentially distributed around the longitudinal axis.

8. The instrument of anyone of claims 5 through 7, **characterized in that** the splines (70) of the first set of splines (70), the splines (72) of the second set of splines (72) and/or the at least one further spline (82) have tapered ends (88).

9. The instrument of anyone of claims 5 through 8, **characterized in that** the first set of splines (70) is arranged adjacent to the second set of splines (72) with no or at least only low frictional contact to one another.

10. The instrument of any one of claims 1 through 5, **characterized in that** the actuating element (52') is rotationally fixedly coupleable with the shaft (12) and/or the elongated element (42) via radially elastically deformable elements (92, 94), a first elastically deformable element (92) being rotationally fixed with respect to the shaft (12) and a second elastically deformable element (94) being rotationally fixed with respect to the elongated element (42).

11. The instrument of claim 10, **characterized in that** the first elastically deformable element (92) and the second elastically deformable element (94) each have at least one leaf spring (98, 110) respectively, extending in longitudinal direction, a respective first end (102) of each leaf spring being (98, 110) fixed with respect to the shaft (12) and the elongated element (42), respectively, and a respective second end (104, 112) of each leaf spring (98, 110) being free and biased in radial direction away from the longitudinal axis (30'), wherein when slid over the respective second end (104, 112) of the respective leaf spring (98, 110), the actuating element (52') urges the respective second end (104, 112) in direction toward the longitudinal axis (30'), the respective second end (104, 112) thereby in rotationally fixed engagement with the actuating element (52').

12. The instrument of claim 11, **characterized in that** the at least one leaf spring (98) of the first elastically deformable element (92) and the at least one leaf spring (110) of the second elastically deformable element (94) are oppositely inclined with respect to the longitudinal axis (30'), wherein the second ends (104, 112) of the leaf springs (98, 110) are facing away from one another.

13. The instrument of claim 11 or 12, **characterized in that** the actuating element (50') has at least one radially inwardly directed protrusion (116, 118) behind which the respective second end (104, 112) of the respective leaf spring (98, 110) comes to lie when in engagement with the actuating element (52').

14. The instrument of any one of claims 11 through 13, **characterized in that** the at least one leaf spring (98) of the first elastically deformable element (92) is in rotationally fixed engagement with the handle (32'), when the actuating element (52') is in the second position, and the at least one leaf spring (110) of the second elastically deformable element (94) is in rotationally fixed engagement with the handle (32'), when the actuating element (52') is in the first position.

15. The instrument of any one of claims 5 through 9, **characterized in that** the elongated element (42) is axially movable with respect to the second set of splines (72).

16. The instrument of any one of claims 10 through 14, **characterized in that** the elongated element (42') is axially movable with respect to the second elastically deformable element (94).

17. The instrument of any one of claims 1 through 16, **characterized in that** the shaft (12; 12') has at least one bent portion (18), which at least one bent portion (18) having at least one fixed curve or at least one articulating joint.

## Patentansprüche

1. Medizinisches Instrument, mit einem langerstreckten Schaft (12; 12'), der ein distales Ende (14) und ein proximales Ende (16; 16') aufweist, einem Arbeitsteil (24), das am distalen Ende (14) des Schaftes (12; 12') angeordnet ist, einem Handgriff (32; 32'), der am proximalen Ende (16; 16') des Schaftes.(12; 12') angeordnet ist, und einem langerstreckten Element (42; 42'), das sich durch den Schaft (12; 12') erstreckt, wobei ein distales Ende (48) des langerstreckten Elements (42; 42') drehfest mit dem Arbeitsteil (24) verbunden ist und ein proximales Ende (44; 44') des langerstreckten Elementes (42; 42') mit dem Handgriff (32; 32') verbunden ist, wobei der Schaft (12; 12') um eine Längsachse (30; 30') des Schaftes (12; 12') drehbar ist, wobei das Arbeitsteil (24) um eine Längsachse (50) des Arbeitsteils (24) unabhängig von dem Schaft (12; 12') relativ zu dem Handgriff (32; 32') drehbar ist, und mit einem Betätigungselement (52; 52'), das an dem Handgriff (32; 32') zum Drehen des Schaftes (12; 12') angeordnet ist, **dadurch gekennzeichnet, dass** das Betätigungselement (52; 52') zwischen zumindest zwei unterschiedlichen axialen Stellungen umschaltbar ist, wobei das Betätigungselement (52; 52') in einer ersten von diesen Stellungen ausschließlich mit dem Schaft (12; 12') zum Drehen nur des Schaftes (12; 12') drehfest gekoppelt ist, und wobei das Betätigungselement (52; 52') in einer zweiten von diesen Stellungen ausschließlich mit dem langerstreckten Element (42; 42') zum Drehen nur des Arbeitsteils (24) drehfest gekoppelt ist.

2. Medizinisches Instrument nach Anspruch 1, **dadurch gekennzeichnet, dass** das Betätigungselement (52; 52') in eine dritte axiale Stellung umschaltbar ist, in der das Betätigungselement (52; 52') gleichzeitig mit dem Schaft (12; 12') und dem langerstreckten Element (42; 42') zum gleichzeitigen Drehen sowohl des Schaftes (12; 12') als auch des Arbeitsteils (24) drehfest gekoppelt ist.

3. Instrument nach Anspruch 1 oder 2, **dadurch gekennzeichnet, dass** das Betätigungselement (52; 52') mit dem Schaft (12; 12') und/oder mit dem langerstreckten Element (42; 42') durch einen formschlüssigen Verriegelungseingriff drehfest koppelbar ist.

4. Instrument nach einem der Ansprüche 1 bis 3, **dadurch gekennzeichnet, dass** das Betätigungselement (52') mit dem Schaft (12') und/oder mit dem langerstreckten Element (42') durch Reibeingriff drehfest gekoppelt ist.

5. Instrument nach einem der Ansprüche 1 bis 4, **dadurch gekennzeichnet, dass** das Betätigungselement (52) mit dem Schaft (12) und/oder dem langerstreckten Element (42) über Sätze von Rippen (70, 72, 82) drehfest koppelbar ist, die sich in Längsrichtung erstrecken, wobei ein erster Satz von Rippen (70) drehfest bezüglich des Schaftes (12), ein zweiter Satz von Rippen (72) drehfest bezüglich des langerstreckten Elementes (42), und zumindest eine weitere Rippe (82) drehfest bezüglich des Betätigungselements (52) ist.

6. Instrument nach Anspruch 5, **dadurch gekennzeichnet, dass** das Betätigungselement (52) mit dem Schaft (12) und/oder dem langerstreckten Element (42) durch axiales Verlagern der zumindest einen weiteren Rippe (82) koppelbar ist, so dass die zumindest eine weitere Rippe (82) mit dem ersten Satz von Rippen (70) und/oder dem zweiten Satz von Rippen (72) kämmt.

7. Instrument nach Anspruch 5 oder 6, **dadurch gekennzeichnet, dass** die Rippen (70) des ersten Satzes von Rippen (70) und die Rippen (72) des zweiten Satzes von Rippen (72) um die Längsachse umfänglich verteilt sind.

8. Instrument nach einem der Ansprüche 5 bis 7, **dadurch gekennzeichnet, dass** die Rippen (70) des ersten Satzes von Rippen (70), die Rippen (72) des zweiten Satzes von Rippen (72) und/oder die zumindest eine weitere Rippe (82) sich verjüngende Enden (88) aufweisen.

9. Instrument nach einem der Ansprüche 5 bis 8, **dadurch gekennzeichnet, dass** der erste Satz von Rippen (70) dem zweiten Satz von Rippen (72) ohne oder mit zumindest nur geringem Reibkontakt aneinander benachbart angeordnet ist.

10. Instrument nach einem der Ansprüche 1 bis 5, **dadurch gekennzeichnet, dass** das Betätigungselement (52') mit dem Schaft (12) und/oder dem langerstreckten Element (42) über radial elastisch verformbare Elemente (92, 94) drehfest koppelbar ist, wobei ein erstes elastisch verformbares Element (92) drehfest bezüglich des Schaftes (12) und ein zweites elastisch verformbares Element (94) drehfest bezüglich des langerstreckten Elements (42) ist.

11. Instrument nach Anspruch 10, **dadurch gekennzeichnet, dass** das erste elastisch verformbare Element (92) und das zweite elastisch verformbare Element (94) jeweils zumindest eine Blattfeder (98, 110) aufweisen, die sich in Längsrichtung erstrecken, wobei ein jeweiliges erstes Ende (102) jeder Blattfeder (98, 110) bezüglich des Schaftes (12) bzw. des langerstreckten Elements (42) fest ist, und wobei ein jeweiliges zweites Ende (104, 112) jeder Blattfeder (98, 110) frei und in radialer Richtung von der Längsachse (30') weg vorgespannt ist, wobei das Betätigungselement (52'), wenn es über das jeweilige zweite Ende (104; 112) der jeweiligen Blattfeder (98, 110) geschoben wird, das jeweilige zweite Ende (104, 112) in Richtung zur Längsachse (30') hin verdrängt, wobei das jeweilige zweite Ende (104, 112) dadurch in drehfesten Eingriff mit dem Betätigungselement (52') ist.

12. Instrument nach Anspruch 11, **dadurch gekennzeichnet, dass** die zumindest eine Blattfeder (98) des ersten elastisch verformbaren Elements (92) und die zumindest eine Blattfeder (110) des zweiten elastisch verformbaren Elements (94) bezüglich der Längsachse (30') entgegengesetzt geneigt sind, wobei die zweiten Enden (104, 112) der Blattfedern (98, 110) voneinander weg weisen.

13. Instrument nach Anspruch 11 oder 12, **dadurch gekennzeichnet, dass** das Betätigungselement (50') zumindest einen radial nach innen gerichteten Vorsprung (116, 118) aufweist, hinter dem das jeweilige zweite Ende (104, 112) der jeweiligen Blattfeder (98, 110) zu liegen kommt, wenn es in Eingriff mit dem Betätigungselement (52') steht.

14. Instrument nach einem der Ansprüche 11 bis 13, **dadurch gekennzeichnet, dass** die zumindest eine Blattfeder (98) des ersten elastisch verformbaren Elementes (92) in drehfestem Eingriff mit dem Handgriff (32') ist, wenn das Betätigungselement (52') in der zweiten Stellung ist, und wobei die zumindest eine Blattfeder (110) des zweiten elastisch verformbaren Elements (94) in drehfestem Eingriff mit dem Handgriff (32') ist, wenn das Betätigungselement (52') in der ersten Stellung ist.

15. Instrument nach einem der Ansprüche 5 bis 9, **dadurch gekennzeichnet, dass** das langerstreckte Element (42) bezüglich des zweiten Satzes von Rippen (72) axial beweglich ist.

16. Instrument nach einem der Ansprüche 10 bis 14, **dadurch gekennzeichnet, dass** das langerstreckte Element (42') bezüglich des zweiten elastisch verformbaren Elements (94) axial beweglich ist.

17. Instrument nach einem der Ansprüche 1 bis 16, **dadurch gekennzeichnet, dass** der Schaft (12; 12') zumindest einen gekrümmten Abschnitt (18) aufweist, wobei der zumindest eine gekrümmte Abschnitt (18) zumindest eine feste Krümmung oder zumindest ein Gelenk aufweist.

## Revendications

1. Instrument médical, comprenant un arbre allongé (12 ; 12') ayant une extrémité distale (14) et une extrémité proximale (16 ; 16'), une partie de travail (24) agencée au niveau de l'extrémité distale (14) de l'arbre (12 ; 12'), une poignée (32 ; 32') agencée au niveau de l'extrémité proximale (16 ; 16') de l'arbre (12 ; 12'), et un élément allongé (42 ; 42') s'étendant à travers l'arbre (12 ; 12'), une extrémité distale (48) de l'élément allongé (42 ; 42') étant reliée en rotation de manière fixe à la partie de travail (24) et une extrémité proximale (44 ; 44') de l'élément allongé (42 ; 42') étant reliée à la poignée (32 ; 32'), l'arbre (12 ; 12') pouvant tourner autour d'un axe longitudinal (30 ; 30') de l'arbre (12 ; 12'), la partie de travail (24) pouvant tourner par rapport à la poignée (32 ; 32') autour d'un axe longitudinal (50) de la partie de travail (24) indépendamment de l'arbre (12 ; 12'), et un élément d'actionnement (52 ; 52') agencé sur la poignée (32 ; 32') pour faire tourner l'arbre (12 ; 12'), **caractérisé en ce que** l'élément d'actionnement (52 ; 52') peut commuter entre au moins deux positions axiales différentes, dans une première position dont l'élément d'actionnement (52 ; 52') est couplé en rotation de manière fixe exclusivement à l'arbre (12 ; 12') pour faire tourner l'arbre (12 ; 12') seulement, et dans une deuxième position dont l'élément d'actionnement (52 ; 52') est couplé en rotation de manière fixe exclusivement à l'élément allongé (42 ; 42') pour faire tourner la partie de travail (24) seulement.

2. Instrument médical de la revendication 1, **caractérisé en ce que** l'élément d'actionnement (52 ; 52') peut commuter dans une troisième position axiale, où l'élément d'actionnement (52 ; 52') est couplé en rotation de manière fixe simultanément à l'arbre (12 ; 12') et à l'élément allongé (42 ; 42') pour faire tourner à la fois l'arbre (12 ; 12') et la partie de travail (24) simultanément.

3. Instrument de la revendication 1 ou 2, **caractérisé en ce que** l'élément d'actionnement (52 ; 52') peut être couplé en rotation de manière fixe à l'arbre (12 ; 12') et/ou à l'élément allongé (42 ; 42') par engagement de verrouillage positif.

4. Instrument de l'une quelconque des revendications 1 à 3, **caractérisé en ce que** l'élément d'actionnement (52') est couplé en rotation de manière fixe à l'arbre (12') et/ou à l'élément allongé (42') par engagement de frottement.

5. Instrument de l'une quelconque des revendications 1 à 4, **caractérisé en ce que** l'élément d'actionnement (52) peut être couplé en rotation de manière fixe à l'arbre (12) et/ou à l'élément allongé (42) via des ensembles de cannelures (70, 72, 82) s'étendant dans une direction longitudinale, un premier ensemble de cannelures (70) étant fixé en rotation par rapport à l'arbre (12), un deuxième ensemble de cannelures (72) étant fixé en rotation par rapport à l'élément allongé (42), au moins une cannelure supplémentaire (82) étant fixée en rotation par rapport à l'élément d'actionnement (52).

6. Instrument de la revendication 5, **caractérisé en ce que** l'élément d'actionnement (52) peut être couplé à l'arbre (12) et/ou à l'élément allongé (42) par déplacement axial de l'au moins une cannelure supplémentaire (82) de sorte que l'au moins une cannelure supplémentaire (82) s'engrène avec le premier ensemble de cannelures (70) et/ou avec le deuxième ensemble de cannelures (72).

7. Instrument de la revendication 5 ou 6, **caractérisé en ce que** les cannelures (70) du premier ensemble de cannelures (70) et les cannelures (72) du deuxième ensemble de cannelures (72) sont distribuées de manière circonférentielle autour de l'axe longitudinal.

8. Instrument de l'une quelconque des revendications 5 à 7, **caractérisé en ce que** les cannelures (70) du premier ensemble de cannelures (70), les cannelures (72) du deuxième ensemble de cannelures (72) et/ou l'au moins une cannelure supplémentaire (82) ont des extrémités effilées (88).

9. Instrument de l'une quelconque des revendications 5 à 8, **caractérisé en ce que** le premier ensemble de cannelures (70) est agencé de manière adjacente au deuxième ensemble de cannelures (72) sans aucun contact de frottement l'un avec l'autre ou avec au moins seulement un faible contact de frottement l'un avec l'autre.

10. Instrument de l'une quelconque des revendications 1 à 5, **caractérisé en ce que** l'élément d'actionnement (52') peut être couplé en rotation de manière fixe à l'arbre (12) et/ou à l'élément allongé (42) via des éléments élastiquement déformables de manière radiale (92, 94), un premier élément élastiquement déformable (92) étant fixé en rotation par rapport à l'arbre (12) et un deuxième élément élastiquement déformable (94) étant fixé en rotation par rapport à l'élément allongé (42).

11. Instrument de la revendication 10, **caractérisé en ce que** le premier élément élastiquement déformable (92) et le deuxième élément élastiquement déformable (94) ont chacun au moins un ressort à lames (98, 110), respectivement, s'étendant dans une direction longitudinale, une première extrémité respective (102) de chaque ressort à lames (98, 110) étant fixée par rapport à l'arbre (12) et à l'élément allongé (42), respectivement, et une deuxième extrémité respective (104, 112) de chaque ressort à lames (98, 110) étant libre et sollicitée dans une direction radiale loin de l'axe longitudinal (30'), où, lors du coulissement sur la deuxième extrémité respective (104, 112) du ressort à lames respectif (98, 110), l'élément d'actionnement (52') pousse la deuxième extrémité respective (104, 112) dans une direction vers l'axe longitudinal (30'), la deuxième extrémité respective (104, 112) étant ainsi en engagement fixe en rotation avec l'élément d'actionnement (52').

12. Instrument de la revendication 11, **caractérisé en ce que** l'au moins un ressort à lames (98) du premier élément élastiquement déformable (92) et l'au moins un ressort à lames (110) du deuxième élément élastiquement déformable (94) sont inclinés de manière opposée par rapport à l'axe longitudinal (30'), où les deuxièmes extrémités (104, 112) des ressorts à lames (98, 110) sont opposées l'une à l'autre.

13. Instrument de la revendication 11 ou 12, **caractérisé en ce que** l'élément d'actionnement (50') a au moins une saillie dirigée radialement vers l'intérieur (116, 118) derrière laquelle la deuxième extrémité respective (104, 112) du ressort à lames respectif (98, 110) vient se placer lors de l'engagement avec l'élément d'actionnement (52').

14. Instrument de l'une quelconque des revendications 11 à 13, **caractérisé en ce que** l'au moins un ressort à lames (98) du premier élément élastiquement déformable (92) est en engagement fixe en rotation avec la poignée (32'), lorsque l'élément d'actionnement (52') est dans la deuxième position, et l'au moins un ressort à lames (110) du deuxième élément élastiquement déformable (94) est en engagement fixe en rotation avec la poignée (32'), lorsque l'élément d'actionnement (52') est dans la première position.

15. Instrument de l'une quelconque des revendications 5 à 9, **caractérisé en ce que** l'élément allongé (42) est axialement mobile par rapport au deuxième ensemble de cannelures (72).

16. Instrument de l'une quelconque des revendications 10 à 14, **caractérisé en ce que** l'élément allongé (42') est axialement mobile par rapport au deuxième élément élastiquement déformable (94).

17. Instrument de l'une quelconque des revendications 1 à 16, **caractérisé en ce que** l'arbre (12 ; 12') a au moins une partie pliée (18), laquelle au moins une partie pliée (18) ayant au moins une courbe fixe ou au moins un joint d'articulation.
